(19) Europäisches Patentamt
European Patent Office
Office européen des brevets

(11) Publication number: **0 289 171 A1**

(12) # EUROPEAN PATENT APPLICATION

(21) Application number: 88303341.7

(51) Int. Cl.$^4$: **C07D 265/24 , A01N 43/86**

(22) Date of filing: 13.04.88

(30) Priority: 14.04.87 US 38090

(43) Date of publication of application:
02.11.88 Bulletin 88/44

(84) Designated Contracting States:
DE FR GB IT

(71) Applicant: UNIROYAL CHEMICAL COMPANY, Inc.
World Headquarters
Middlebury Connecticut 06749(US)

Applicant: UNIROYAL CHEMICAL LTD./UNIROYAL CHEMICAL LTEE
25 Erb Street
Elmira Ontario N3B 3A3(CA)

(72) Inventor: Brouwer, Walter Gerhard
15 Hickory Street
Guelph Wellington Ontario, N1G 2X2(CA)
Inventor: Felauer, Ethel Ellen
RR 1, Puslinch
Wellington Ontario, N0B 2JO(CA)
Inventor: Moore, Richard Charles
5 Jones Road
Wallingford New Haven Connecticut
06791(US)

(74) Representative: Harrison, Michael Robert et al
Urquhart-Dykes & Lord 91 Wimpole Street
London W1M 8AH(GB)

(54) **Substituted 2H-benzoxazin-2-ones.**

(57) A new class of N-alkyl or N-alkenyl, 2,3-dihydro-4-thioxo-2H-1,3-benzoxazin-2-ones is disclosed. This class of compounds is useful in the control of plant pests. Thus, pesticidal compositions comprising these benzoxazin-2-ones with a carrier therefor are also described. In addition, a process for forming these benzoxazin-2-one compounds is also set forth.

EP 0 289 171 A1

# SUBSTITUTED 2H-BENZOXAZIN-2-ONES

## BACKGROUND OF THE DISCLOSURE

### 1. Field of the Invention

The instant invention is directed to a new class of substituted 2H-benzoxazin-2-one compounds. More specifically, the present invention is directed to a new class of substituted 2H-benzoxazin-2-one compounds useful as pesticides.

### 2. Background of the Prior Art

The devastation caused by harmful plant pests, especially insects, nematodes and arachnids, represents a serious economic threat to commercially important food, fiber and ornamental plants. For this reason the development of new, more effective pesticides to control these plant pests represents a continuous ongoing scientific activity.

Benzoxazine derivative compounds are known in the art. For example, British Patent Application 2,031,410 (Chemical Abstract 94:121560g) discloses a new class of compounds possessed of analgesic and anti-inflammatory activity. Japanese Patent Publication 76-4,186 (Chemical Abstract 85:21390x) sets forth a class of 2,3-dihydro-4H-1,3-benzoxazines. These compounds are claimed to be effective against rice blast. R. Bindra et al., Indian J. Pharm. 37, 133 (1975) (Chemical Abstract 84:105512f) describes a class of benzoxazines, specifically, 4-oxo-2,3-dihydro-1,3-benzoxazine compounds, which exhibit bacteriacidal and fungicidal activity. Finally, E.S. Charles et al., Arch. Pharm. 315, 97 (1982) (Chemical Abstract 96:217779c) describes the synthesis of benzoxazines, among other classes of compounds, which were screened for antihelmintic and antimicrobial activity. Most of these compounds were found to be inactive.

None of the compounds disclosed in any of the above discussed references define an N-alkyl or N-alkenyl-2,3-dihydro-4-thioxo-2H-1,3-benzoxazin-2-one compound. However, compounds having structural formulas similar to such compounds, albeit not N-alkyl or N-alkenyl benzoxazin-2-ones, have been synthesized by thiolation of the corresponding 2,4-dione to produce mixtures of thiolated compounds. That is, thiolation reactions in the prior art have resulted in the formation of mixtures of 2,3-dihydro-4-dioxo-2H-1,3-benzoxazin-2-ones and 2,3-dihydro-2,4-dithione-2H-1,3-benzoxazines. Such a teaching is made in G. Wagner et al., Pharmazie 21, 161 (1966), (Chemical Abstract 65:8900).

Using this method G. Wagner et al., Z. Chem. 2, 306 (1962) (Chemical Abstract 58:12460e) have isolated from such a mixture the compound 2,3-dihydro-3-methyl-4-thioxo-2H-1,3-benzoxazin-2-one. This paper provides no disclosure of utility of this compound or any other compound recited therein. The paper is solely directed to a general method for preparing 2-hydroxythiobenzamides.

Of interest are organophosphorus compounds which are effective insecticides and miticides possessing low toxicity to warmblooded animals as well as low residual toxicity. Such compounds are formed by reacting substituted 1,3-benzoxazines with a halogenated organophosphorus compound. These compounds are 2,4-dionebenzoxazines wherein an organophosphorus radical is bonded to the heterogeneous nitrogen atom. Disclosure of compounds of this kind is made in U.S. Patent 3,467,655 and Japanese Patent Publication 44-12145.

The above remarks establish that the art has not recognized the potential of substituted N-alkyl or N-alkenyl 2H-1,3-benzoxazin-2-ones. That only one such compound is known to exist in the prior art establishes the novelty of these compounds and the novelty of using this class of compounds in the control of plant pests. This lack of appreciation may also be due to the difficulty in forming such compounds in the prior art. The Wagner et al. papers, discussed above, teach a method wherein the desired product is formed as one of at least two classes of compound products. In the prior art, the separation of the desired compounds is expensive, time consuming and difficult.

## SUMMARY OF THE INVENTION

It has now been discovered that a new class of N-alkyl and N-alkenyl 2H-1,3-benzoxazin-2-ones, a class of compounds substantially unknown in the prior art, can easily be synthesized, without costly and difficult separation operations, to produce a class of compounds effective against many insects, arachnids and nematodes which attack economically important plants and crops.

In accordance with the present invention a compound having the structural formula

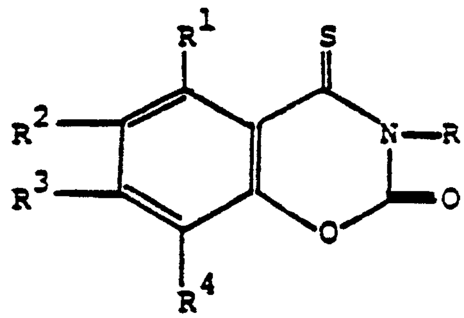

where R is $C_1$-$C_6$ alkyl or $C_2$-$C_6$ alkenyl; $R^1$ is hydrogen or $C_1$-$C_6$ alkyl; $R^2$ is hydrogen, $C_1$-$C_6$ alkyl, $C_1$-$C_6$ alkoxy, halogen, alkylthio, $C_3$-$C_6$ cycloalkyl, $C_7$-$C_9$ aralkyl, $C_7$-$C_9$ aralkyloxy, carboxy or $C_2$-$C_5$ carbalkoxy; $R^3$ is hydrogen, $C_1$-$C_6$ alkyl, $C_1$-$C_6$ alkoxy, halogen, $C_6$-$C_{10}$ aryl or $C_1$-$C_2$ haloalkyl; and $R^4$ is hydrogen $C_1$-$C_6$ alkyl, $C_1$-$C_6$ alkoxy, halogen, $C_7$-$C_9$ aralkyl or $C_7$-$C_9$ aralkyloxy with the proviso that R cannot be methyl if $R^1$, $R^2$, $R^3$ and $R^4$ are each hydrogen.

In further accordance with the instant invention a process is provided for forming compounds having the structural formula

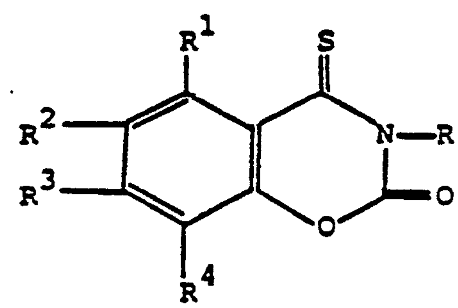

where are R is $C_1$-$C_6$ alkyl or $C_2$-$C_6$ alkenyl; $R^1$ is hydrogen or $C_1$-$C_6$ alkeny; $R^2$ is hydrogen, $C_1$-$C_6$ alkyl, $C_1$-$C_6$ alkoxy, halogen, alkylthio, $C_3$-$C_6$ cycloalkyl, $C_7$-$C_9$ aralkyl, $C_7$-$C_9$ aralkyloxy, carboxy or $C_2$-$C_5$ carbalkoxy; $R^3$ is hydrogen, $C_1$-$C_6$ alkyl, $C_1$-$C_6$ alkoxy, halogen, $C_6$-$C_{10}$ aryl, $C_1$-$C_2$ haloalkyl; and $R^4$ is hydrogen, $C_1$-$C_6$ alkyl, $C_1$-$C_6$ alkoxy, halogen, $C_7$-$C_9$ aralkyl or $C_7$-$C_9$ aralkyloxy comprising reacting, at a temperature of up to 100°C, a compound having the structural formula

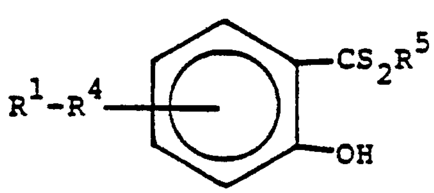

where $R^5$ is $C_1$-$C_6$ alkyl; and $R^1$, $R^2$, $R^3$ and $R^4$ have the meanings given above with a compound having the structural formula RNCO, where R has the meanings given above, in the presence of an organic base.

In further accordance with the instant invention a process if provided for controlling plant pests which comprises applying to the locus to be protected a pesticidally effective amount of a compound having the structural formula

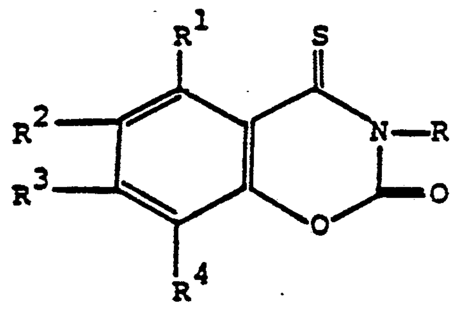

where R is $C_1$-$C_6$ alkyl or $C_2$-$C_6$ alkenyl; $R^1$ is hydrogen or $C_1$-$C_6$ alkyl $R^2$ is hydrogen, $C_6$-$C$· alkyl, $C_1$-$C_6$ alkoxy, halogen, alkylthio, $C_3$-$C_6$ cycloalkyl, $C_7$-$C_9$ aralkyl, $C_7$-$C_9$ aralkyloxy, carboxy or $C_2$-$C_5$ carbalkoxy; $R^3$ is hydrogen, $C_1$-$C_6$ alkyl, $C_1$-$C_6$ alkoxy, halogen, $C_6$-$C_{10}$ aryl or $C_1$-$C_2$ haloalkyl; and $R^4$ is hydrogen, $C_1$-$C_6$ alkyl, $C_1$-$C_6$ alkoxy, halogen, $C_7$-$C_9$ aralkyl or $C_7$-$C_9$ aralkyloxy.

In still further accordance with the present invention a pesticidal composition is provided. The pesticidal composition comprises a compound having the structural formula

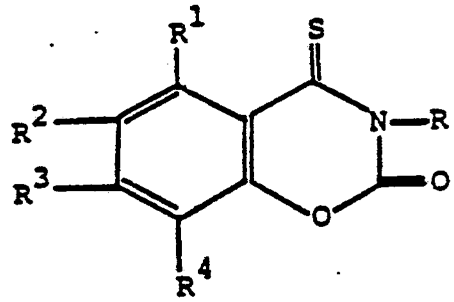

where R is $C_1$-$C_6$ alkyl or $C_2$-$C_6$ alkenyl; $R^1$ is hydrogen or $C_1$-$C_6$ alkyl; $R^2$ is hydrogen, $C_1$-$C_6$ alkyl, $C_1$-$C_6$ alkoxy, halogen, alkylthio, $C_3$-$C_6$ cycloalkyl, $C_7$-$C_9$ aralkyl, $C_7$-$C_9$ aralkyloxy, carboxy or $C_2$-$C_5$ carbalkoxy; $R^3$ is hydrogen, $C_1$-$C_6$ alkyl, $C_1$-$C_6$ alkoxy, halogen, $C_6$-$C_{10}$ aryl or $C_1$-$C_2$ haloalkyl; and $R^4$ is hydrogen, $C_1$-$C_6$ alkyl, $C_1$-$C_6$ alkoxy, halogen, $C_7$-$C_9$ aralkyl or $C_7$-$C_9$ aralkyloxy and a carrier therefor.

## DETAILED DESCRIPTION

The present invention is directed to a class of compounds having the stuctural formula

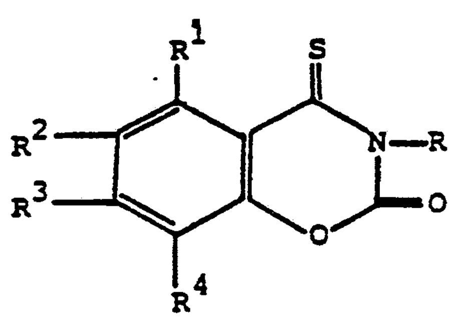

I

where R is $C_1$-$C_6$ alkyl or $C_2$-$C_6$ alkenyl; $R^1$ is hydrogen or $C_1$-$C_6$ alkyl; $R^2$ is hydrogen, $C_1$-$C_6$ alkyl, $C_1$-$C_6$ alkoxy, halogen, alkylthio, $C_3$-$C_6$ cycloalkyl, $C_7$-$C_9$ aralkyl, $C_7$-$C_9$ aralkyloxy, carboxy or $C_2$-$C_5$ carbalkoxy; $R^3$ is hydrogen, $C_1$-$C_6$ alkyl, $C_1$-$C_6$ alkoxy, halogen, $C_6$-$C_{10}$ aryl or $C_1$-$C_2$ haloalkyl; and $R_4$ is hydrogen, $C_1$-$C_6$ alkyl, $C_1$-$C_6$ alkoxy, halogen, $C_7$-$C_9$ aralkyl or $C_7$-$C_9$ aralkyloxy with the proviso that R cannot be methyl if $R^1$, $R^2$, $R^3$ and $R^4$ are each hydrogen.

More preferably, the present invention is directed to a class of compounds having the structural formula I where R is $C_1$-$C_4$ alkyl or $C_2$-$C_4$ alkenyl; $R^1$ is hydrogen or $C_1$-$C_4$ alkyl; $R^2$ is hydrogen, $C_1$-$C_4$ alkyl, $C_2$-$C_4$

carbalkoxy, $C_1$-$C_4$ alkoxy or halogen; $R^3$ is hydrogen, $C_1$-$C_4$ alkyl, $C_1$-$C_4$ alkoxy or trifluoromethyl; and $R^4$ is hydrogen, $C_1$-$C_4$ alkyl, $C_1$-$C_4$ alkoxy, $C_7$-$C_8$ aralkyl or halogen with the proviso that R cannot be methyl if $R^1$, $R^2$, $R^3$ and $R^4$ are each hydrogen.

Most preferably, the present invention is directed to a class of compounds having the structural formula I where R is $C_1$-$C_2$ alkyl; $R^1$ is hydrogen; $R^2$ is hydrogen or methyl; $R^3$ is hydrogen, $C_1$-$C_3$ alkyl or $C_1$-$C_3$ alkoxy; and $R^4$ is hydrogen or $C_1$-$C_4$ alkyl with the proviso that R cannot be methyl if $R^1$, $R^2$, $R^3$ and $R^4$ are each hydrogen.

Another aspect of the present invention involves a process for forming the compound having the structural formula denoted as I where R is $C_1$-$C_6$ alkyl or $C_2$-$C_6$ alkenyl; $R^1$ is hydrogen or $C_1$-$C_6$ alkenyl $R^2$ is hydrogen, $C_1$-$C_6$ alkyl, $C_1$-$C_6$ alkoxy, halogen, alkylthio, $C_3$-$C_6$ cycloalkyl, $C_7$-$C_9$ aralkyl, $C_7$-$C_9$ aralkyloxy, carboxy or $C_2$-$C_5$ carbalkoxy; $R_3$ is hydrogen, $C_1$-$C_6$ alkyl, $C_1$-$C_6$ alkoxy, halogen, $C_6$-$C_{10}$ aryl or $C_1$-$C_2$ haloalkyl; and $R^4$ is hydrogen, $C_1$-$C_6$ alkyl, $C_1$-$C_6$ alkoxy, halogen, $C_7$-$C_9$ aralkyl or $C_7$-$C_9$ aralkyloxy. In this process a 2-hydroxybenzenecarbodithioate ester having the structural formula

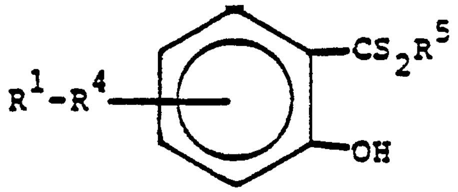

II

where $R^5$ is $C_1$-$C_6$ alkyl; and $R^1$, $R^2$, $R^3$, and $R^4$ have the meanings given above, is reacted with an isocyanate compound having the structural formula RNCO, where R has the meanings given above, at a temperature of up to 100°C in the presence of an organic base to form the compound having the structural formula I. Preferably, the reaction occurs at the reflux temperature of the reaction. Although any organic base can be employed, in a preferred embodiment the organic base employed is pyridine or triethylamine.

The ester having the structural formula II is itself the product of a three-step reaction wherein a benzaldehyde having the structural formula

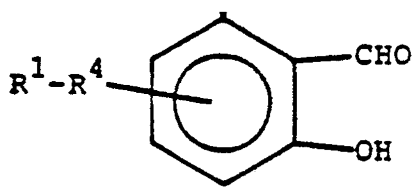

III

where $R^1$, $R^2$, $R^3$ and $R^4$ have the meanings given above is reacted with a cyclic secondary amine, and a source of sulfur. Preferably, the amine is morpholine or piperidine. The source of sulfur is preferably elemental sulfur. The product of this reaction is a compound having the structural formula

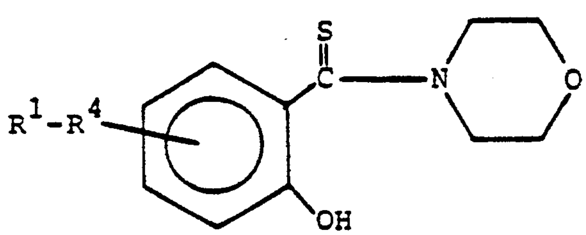

IV

where $R^1$, $R^2$, $R^3$, and $R^4$ have the meanings given above. The compound having the structural formula IV is then reacted with a halide having the structural formula $R^5X$ where $R^5$ has the meanings given earlier; and X is halogen. More preferably, $R^1$ is $C_1$-$C_4$ alkyl and X is iodine. Most preferably, the compound $R^1X$ is methyl iodide. The product of this reaction is a compound having the structural formula

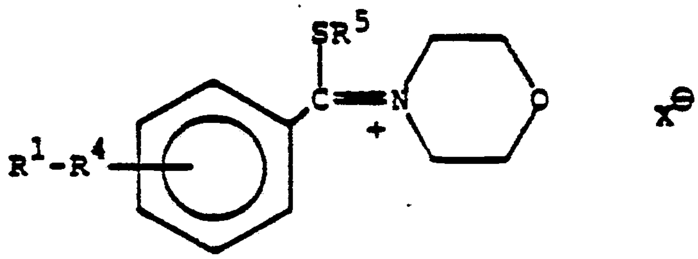

V

where R, $R^1$, $R^2$, $R^3$, and $R^4$ have the meanings given above;

In the last step of the process the compound having the structural formula V is reacted with hydrogen sulfide in the presence of an organic base, preferably pyridine, to produce the 2-hydroxybenzenecarbodithioate ester having the structural formula II.

Another aspect of the subject invention is directed to a process for controlling plant pests. In this process a pesticidally effective amount of a compound having the structural formula I where R is $C_1$-$C_6$ alkyl or $C_2$-$C_6$ alkenyl; $R^1$ is hydrogen or $C_1$-$C_6$ alkyl; $R^2$ is hydrogen, $C_1$-$C_6$ alkyl, $C_1$-$C_6$ alkoxy, halogen, alkylthio, $C_3$-$C_6$ cycloalkyl, $C_7$-$C_9$ aralkyl, $C_7$-$C_9$ aralkyloxy, carboxy or $C_2$-$C_5$ carbalkoxy; $R^3$ is hydrogen, $C_1$-$C_6$ alkyl, $C_1$-$C_6$ alkoxy, halogen, $C_6$-$C_{10}$ aryl or $C_1$-$C_2$ haloalkyl; and $R^4$ is hydrogen, $C_1$-$C_4$ alkyl, $C_1$-$C_6$ alkoxy, halogen, $C_7$-$C_9$ aralkyl or $C_7$-$C_9$ aralkyloxy is applied to the locus to be protected.

More preferably, the process for controlling plant pests involves applying to the locus to be protected a pesticidally effective amount of the compound having the structural formula I wherein R is $C_1$-$C_4$ alkyl or $C_1$-$C_6$ alkenyl; $R^1$ is hydrogen or $C_1$-$C_4$ alkyl; $R^2$ is hydrogen, $C_1$-$C_6$ alkyl, $C_2$-$C_4$ carbalkoxy, $C_1$-$C_4$ alkoxy or halogen; $R^3$ is hydrogen, $C_1$-$C_4$ alkyl, $C_1$-$C_4$ alkoxy or trifluoromethyl; and $R^4$ is hydrogen, $C_1$-$C_4$ alkyl, $C_1$-$C_4$ alkoxy, $C_7$-$C_8$ aralkyl or halogen.

Still more preferably, the process for controlling pests involves applying to the locus to be protected a pesticidally effective amount of a compound having structural formula I wherein R is $C_1$-$C_2$ alkyl; $R^1$ is hydrogen; $R^2$ is hydogen or methyl; $R^3$ is hydrogen, $C_1$-$C_3$ alkyl or $C_1$-$C_3$ alkoxy; and $R^4$ is hydrogen or $C_1$-$C_4$ alkyl.

The plant pests controlled in this aspect of the invention are insects, nematodes and arachnids. Of the insects controlled by the present invention, tobacco budworm, corn rootworm and rice planthoppers are especially vulnerable to the compounds of the present invention. Of the arachnids, mites are effectively controlled by the compounds of the present invention.

The last aspect of the instant invention is directed to a composition for the control of plant pests which comprise a compound having the structural formula I where R is $C_1$-$C_6$ alkyl or $C_2$-$C_6$ alkenyl; $R^1$ is hydrogen or $C_1$-$C_6$ alkyl; $R^2$ is hydrogen, $C_1$-$C_6$ alkyl, $C_1$-$C_6$ alkoxy, halogen, alkylthio, $C_3$-$C_6$ cycloalkyl, $C_7$-$C_9$ aralkyl, $C_7$-$C_9$ aralkyloxy, carboxy or $C_2$-$C_5$ carbalkoxy; $R^3$ is hydrogen, $C_1$-$C_6$ alkyl, $C_1$-$C_6$ alkoxy, halogen, $C_6$-$C_{10}$ aryl or $C_1$-$C_2$ haloalkyl; and $R^4$ is hydrogen, $C_1$-$C_6$ alkyl, $C_1$-$C_6$ alkoxy, halogen, $C_7$-$C_9$ aralkyl or $C_7$-$C_9$ aralkyloxy and a carrier therefor.

More preferably, the pesticidal composition of the subject invention is a compound having the structural formula I where R is $C_1$-$C_4$ alkyl or $C_2$-$C_4$ alkenyl; $R^1$ is hydrogen or $C_1$-$C_4$ alkyl; $R^2$ is hydrogen, $C_1$-$C_4$ alkyl, $C_2$-$C_4$ carbalkoxy, $C_1$-$C_4$ alkoxy or halogen; $R^3$ is hydrogen, $C_1$-$C_4$ alkyl, $C_1$-$C_4$ alkoxy or trifluoromethyl; and $R^4$ is hydrogen, $C_1$-$C_4$ alkyl, $C_1$-$C_4$ alkoxy, $C_7$-$C_8$ aralkyl or halogen and a carrier therefor.

Still more preferably, the pesticidal composition of the present invention includes a compound having the structural formula I where R is $C_1$-$C_2$ alkyl; $R^1$ is hydrogen; $R^2$ is hydrogen or methyl; $R^3$ is hydrogen, $C_1$-$C_3$ alkyl or $C_1$-$C_3$ alkoxy; and $R^4$ is hydrogen or $C_1$-$C_4$ alkyl and a carrier therefor.

In a preferred embodiment the composition of the present invention is an insecticidal composition. In another preferred embodiment, the composition of the present invention is a nematocidal composition. In still another preferred embodiment of the present invention the composition is a miticidal composition.

The composition of the present invention includes a carrier. The carrier, within the contemplation of this invention, may be a finely divided or a granular organic or inorganic inert material. Among the inert carriers within the contemplation of this invention are attapulgite clay, sand, vermiculite, corncobs, activated carbon and mineral silicates such as mica, talc, pyrophyllite and clays.

In another embodiment of the composition of this invention the composition comprises a solution. That is, the compound having the structural formula I is dissolved in a suitable solvent which acts as the carrier. Among the solvents within the contemplation of the carrier of the composition of this invention are acetone, methanol, isopropanol, t-butyl alcohol, cyclohexanol, n-butyl alcohol, cyclohexanone, toluene, xylene, dioxane, dimethylformamide, dimethylsulfoxide, ethylene dichloride, and N-methylpyrrolidone.

In still another preferred embodiment of the composition of this invention, the composition comprises a water emulsion carrier. The emulsion is prepared from a solution as described in the previous paragraph. To the solution is added a surface active agent. Surface active agents suitable for use in forming the emulsion of this invention are known to those skilled in the art. McCutcheon's Detergents and Emulsifiers, Allured Publishing Corp., Ridgewood, N.J. (1970); U.S. Patent 2,514,916, Columns 2-4; and U.S. Patent 2,547,734, Columns 3 and 4 provide detailed examples of such surface active agents. These surface active agents may be anionic, non-anionic or cationic.

In yet still another preferred embodiment of the composition of this invention, the composition includes a dispersant as the carrier. In this embodiment the active agent, the compound having the structural formula I, is mixed with a surface active agent of the type described above. Water is then added to form a dispersion. The amount of water added determines the concentration of the composition. The surface active agent may be added neat, that is, as a pure compound, or may be premixed with a solvent of the type described above to form a solution which is added to the surface active agent and water.

In still another embodiment of the composition of this invention the active compound, the compound having the structural formula I, is premixed with an inert solid carrier which is added to a surface active agent and water to provide yet another form of dispersion within the contemplation of the composition of this invention.

The embodiment discussed immediately above, the disposition of the active compound of this invention on a solid inert carrier, which is dispersed to form a dispersion, may alternately be employed in non-liquid form. That is, the composition of this invention may take the form of a dust, granules, a paste or a wettable powder. In these embodiments compound I, the active agent, is admixed with the inert carrier to form a solid composition. For example, in a preferred embodiment wherein a powdered composition is formed, the solid inert carrier is provided as a powder . The inert carrier in this application is a mineral silicate. This solid may be made wettable by the addition of a surface active agent, well known to those skilled in the art, and referred to in the above-recited references directed to surface active agents.

In yet still another principal embodiment of the composition of this invention, an aerosol is prepared. To prepare the aerosol, the active compound is dissolved in a first solvent. This first solvent is conventional in the sense that although it is volatile it is not highly volatile. The thus formed solution is then admixed with a highly volatile solvent, a so-called liquid aerosol carrier. The aerosol carrier is liquid only under elevated pressure. At ambient temperature and pressure the aerosol carrier is a gas. In a sub-embodiment of this aerosol embodiment, the aerosol carrier may itself be active. For example, the aerosol may be an insecticide, a herbicide, a bactericide or a plant growth regulant.

The following examples are given to illustrate the scope of the present invention. Because these examples are given for illustrative purposes only, the invention embodied therein should not be limited to the actual examples provide.

## EXAMPLE 1

### Preparation of 3,4-Dihydro-3-methyl-8-(1-methylethyl)-4-thioxo-2H-1,3-benzoxazin-2-one (Compound No 15)

2-Isopropylphenol (94 g., 0.7 mol) dissolved in tetrahydrofuran was added slowly to a Grignard reagent solution obtained by adding ethyl bromide (100g.,0.9 mol) and magnesium (18.8 g.) in tetrahydrofuran. After removal of the solvent, dry toluene (200 ml.) was added and it too was removed. Toluene (200 ml.) was added to the residue followed by the addition thereto of 143 g. of hexamethylphosphoramide and the cautious addition of paraformaldehyde (60 g., 2 mol) in portions. The reaction mixture was then stirred and refluxed for 6.5 hours. A homogeneous pale yellow solution was obtained. After the solution was cooled to ambient temperature the solution was washed with 10 percent aqueous hydrochloric acid, then water, dried and evaporated to leave a residual oil which was distilled to give 48.6 g. of 2-hydroxy-3-(1-methylethyl)-benzaldehyde having a boiling point of 60°-65°C at 0.25 mm. Hg.

The benzaldehyde (48.6 g., 0.3 mol) thus obtained was mixed with sulfur (11.2 g., 0.35 mol) in morpholine (200 ml.) and slowly heated to 120°C. It was maintained at this temperature for 3 hours and then cooled to ambient temperature. The resultant product was put in ice water thus obtaining a two-phase liquid. The oil phase was separated from the two-phase liquid. The oil phase, on standing, solidified. The solid was collected, washed with water and dried to yield 68 g. of product. A portion of the product was recrystallized from isopropyl alcohol to give 4-[[2-hydroxy-3-(1-methylethyl)phenyl]thioxomethyl]morpholine.

The thus formed morpholide had a melting point of 121-123°C. An elemental analysis yield the following result: (Found C, 64.03; H, 7.63; N, 5.10; $C_{14}H_{19}NO_2S$ requires C, 3.40; H, 7.17; N, 5.28).

This morpholide (63 g., 0.24 mol) was dissolved in acetone (300 ml.). The solution was stirred overnight at ambient temperature with methyl iodide (40 g.). A pale, yellow precipitate was formed and collected on a filter. The precipitate was washed with acetone and dried. The product, 4-[[2-hydroxy-3-(1-methylethyl)-phenyl](methylthio)methylene]morpholinium iodide was obtained in the yield of 65 g. The iodide was isolated and an elemental analysis conducted (Found: C, 44.90; H, 5.87; N, 3.60; $C_{15}H_{22}INO_2S$ requires C, 44.22; H, 5.41; N, 3.44).

The morpholinium iodide salt (60 g., 0.15 mol) was added in portions to pyridine (200 ml.) into which hydrogen sulfide was continuously bubbled. After this addition, the reaction was warmed to 60°C for 3 hours with hydrogen sulfide still being bubbled into the reaction. A dark amber oil was precipitated when the reaction mass was added to an ice/dilute hydrochloric acid slurry. The product of this reaction was extracted with methylene chloride, washed with water and the solvent removed to yield 25 g. of crude product which gave 12 g. of methyl 2-hydroxy-3-(1-methylethyl)benzenecarbodithioate upon distillation at a temperature of 128°-132°C and a pressure of 0.25mm. Hg.

The thus formed benzenecarbodithioate (6.8 g.,0.03 mol) in triethylamine (15 ml.) is refluxed with methyl isocyanate (2.3 g., 0.04 mol) for one hour. After the removal of the solvent and the taking up of the residual red oil in isopropyl alcohol, a yellow precipitate was obtained upon cooling to 0°C. The product obtained, 3,4-dihydro-3-methyl-8-(1-methylethyl)-4-thioxo-2H-1,3-benzoxazin-2-one was obtained in the yield of 4.3 g. The product had a melting point of 86°-87°C and the result of an elemental analysis was as follows: (Found: C, 61.13; H, 5.57; N, 5.94, $C_{12}H_{13}NO_2S$ requires C, 61.28; H, 5.53; N, 5.96).

## EXAMPLE 2

### Preparation of 3,4-Dihydro-3,6-dimethyl-4-thioxo-2H-1,3-benzoxazin-2-one (Compound No. 7)

Methyl 2-hydroxy-5-methylbenzenecarbodithioate (19.8 g., 0.1 mol) and methyl isocyanate (5.7 g., 0.1 mol) in triethylamine (30 ml.) was stirred together whereupon an exotherm developed and a yellow precipitate formed. This precipitate (9 g.) was collected by filtration, washed with toluene and dried. It was characterized by a melting point of 149°-151°C.

The filtrate thus obtained was treated with additional methyl isocyanate (6 g.) and stirred overnight at ambient temperature. Removal of the solvent gave 4 g. of the product, 3,4-dihydro-3,6-dimethyl-4-thioxo-2H-1,3-benzoxazin-2-one. This product, purified from isopropyl alcohol, was obtained in the yield of 4 g. It was characterized by a melting point of 148°-150°C. An elemental analysis conducted on the product gave the following result: (Found: C, 57.76; H, 4.34: N, 6.79. $C_{10}H_9NO_2S$ requires C, 57.97; H, 4.35; N, 6.76).

## EXAMPLE 3

### Preparation of 3,4-Dihydro-3-(2-propenyl)-4-thioxo-2H-1,3-benzoxazin-2-one (Compound No. 4)

Methyl 2-hydroxybenzenecarbodithioate (9 g., 0.05 mol) and allyl isocyanate (4,1 g., 0.05 mol) in triethylamine (25 ml.) was stirred together whereupon an exotherm developed. After refluxing for two hours, the solvent was removed and the residue dissolved in ether. The ether extract was washed in a sodium hydroxide solution, then with water and dried. Removal of the ether yielded a yellow solid. Crystallization of the solid from a mixture of .ether and a low boiling petroleum ether yielded 5 g. of 3,4-dihydro-3-(2-propenyl)-4-thioxo-2H-1,3-benzoxazin-2-one.

This product had a melting point of 63°-64°C. An elemental analysis conducted yielded the following: (Found: C, 59.67; H, 4.02; N, 6.43. $C_{11}H_9NO_2S$ requires C, 60.27; H, 4.14; N, 6.39).

## EXAMPLE 4

Preparation of 3,4-Dihydro-4-thioxo-3,7,8-trimethyl-2H-1,3-benzoxazin-2-one (Compound No. 29)

Methyl 2-hydroxy-3,4-dimethylbenzenecarbodithioate (6.4 g., 0.3 mol) in pyridine (16 ml.) and triethylamine (4 ml.) was treated with methyl isocyanate (3 g., 0.06 mol) and the mixture refluxed for three hours. After removal of the solvent the residue was recrystallized from isopropyl alcohol to yield 4,4 g. of yellow needles of 3,4-dihydro-4-thioxo-3,7,8-trimethyl-2H-1,3-benzoxazin-2-one.

The product was analyzed and found to have a melting point of 176°-178°C. An elemental analysis yielded the following: (Found: C, 60.34; H, 5.00, N, 6.49. $C_{11}H_{11}NO_2S$ requires C, 59.73; H, 4.98; N, 6.33).

EXAMPLE 5

Preparation of Compound Nos. 1-3, 5, 6, 8-14, 16-28 and 30-37

Additional compounds were prepared using essentially the process described in Example 1. In those cases where the starting compounds were not commercially available they were synthesized by methods well known in the art.

These compounds, Compound Nos. 1, 3, 5, 6, 8-14, 16-28 and 30-37, as well as Compound Nos. 4, 7, 15 and 29, the compounds synthesized in Examples 1, 2, 3 and 4, respectively, are summarized in Table I below. Each of these compounds, Compound Nos. 1-37, are characterized in Table I by their melting points.

TABLE 1

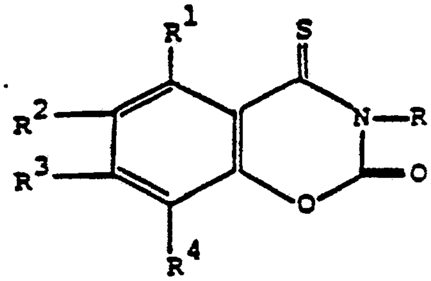

| Cpd. No. | R | $R^1$ | $R^2$ | $R^3$ | $R^4$ | M.P., °C |
|---|---|---|---|---|---|---|
| 1 | $CH_3$ | H | H | H | H | 118-119 |
| 2 | $CH_2CH_3$ | H | H | H | H | 106-108 |
| 3 | $CH(CH_3)_2$ | H | H | H | H | 115-116 |
| 4 | $CH_2CH=CH_2$ | H | H | H | H | 63-64 |
| 5 | $CH_3$ | H | Br | H | H | 205-206 |
| 6 | $CH_3$ | H | $OCH_3$ | H | H | 148-149 |
| 7 | $CH_3$ | H | $CH_3$ | H | H | 148-150 |
| 8 | $CH_3$ | H | F | H | H | 115-116 |
| 9 | $CH_3$ | H | $OC_4H_9$ | H | H | 100-101 |
| 10 | $CH_3$ | H | $CO_2CH_3$ | H | H | 180-181 |
| 11 | $CH_3$ | H | $CH(CH_3)_2$ | H | H | 80-82 |
| 12 | $CH_3$ | H | $SC_3H_7$ | H | H | 104-106 |
| 13 | $CH_3$ | H | H | H | $CH_3$ | 140-141 |
| 14 | $CH_3$ | H | H | H | $C(CH_3)_3$ | 98-99 |
| 15 | $CH_3$ | H | H | H | $CH(CH_3)_2$ | 86-87 |
| 16 | $CH_3$ | H | H | H | $OCH_3$ | 200-201 |
| 17 | $CH_3$ | H | H | H | $CH(CH_3)C_2H_5$ | 78-79 |
| 18 | $CH_3$ | H | H | H | $CH_2C_6H_5$ | 91-92 |
| 19 | $CH_3$ | H | H | H | F | 142-144 |
| 20 | $CH_3$ | H | H | H | $OCH(CH_3)_2$ | 100-102 |
| 21 | $CH_3$ | H | H | H | $OCH_2C_6H_5$ | 147-148 |
| 22 | $CH_3$ | H | H | $OCH_3$ | H | 176-177 |

**TABLE I (continued)**

| Cpd. No. | R | $R^1$ | $R^2$ | $R^3$ | $R^4$ | M.P., °C |
|---|---|---|---|---|---|---|
| 23 | $CH_3$ | H | H | $CH_3$ | H | 164-165 |
| 24 | $CH_3$ | H | H | $CH(CH_3)_2$ | H | 69-70 |
| 25 | $CH_3$ | H | H | Br | H | 180-182 |
| 26 | $CH_3$ | H | H | $OCH(CH_3)_2$ | H | 133-135 |
| 27 | $CH_3$ | H | H | $CF_3$ | H | 143-145 |
| 28 | $CH_3$ | H | H | $C_6H_5$ | H | 148-149 |
| 29 | $CH_3$ | H | H | $CH_3$ | $CH_3$ | 176-178 |
| 30 | $CH_3$ | H | $C_6H_{12}$ | H | H | 140-141 |
| 31 | $CH_3$ | H | $OCH_2C_6H_5$ | H | H | 103-104 |
| 32 | $CH_3$ | H | $CH_2C_6H_5$ | H | H | 147-149 |
| 33 | $CH_3$ | H | H | H | Br | 127-129 |
| 34 | $CH_2CH_3$ | H | $CH_3$ | $CH_3$ | H | 62-65 |
| 35 | $CH_3$ | H | $CH_3$ | $CH_3$ | H | 156-157 |
| 36 | $CH_3$ | H | H | $(CH_3)_3C$ | H | 120-122 |
| 37 | $CH_2CH_3$ | H | H | $(CH_3)_3C$ | H | 96-98 |

## EXAMPLE 6

### Control of Tobacco Budworm

Compound Nos. 1-37, representative of the benzoxazin-2-one compounds of the present invention, were tested to determine their effectiveness in controlling the insect, tobacco budworm, that is, the species *Heliothis virescens*, an important threat to tobacco crops. To determine the effectiveness of the compounds of the present invention these compounds were each prepared in a concentration of 3,000 parts per million (ppm) by dissolving 0.3 g of each of the compounds in 10 ml. of acetone to which 4 drops of a wetting agent, an aqueous solution (0.05 ppm) of polyoxyethylene sorbitan. This solution was diluted with water until a dispersion of 100 ml. was formed. This dispersion yielded the 3,000 ppm of each of the compounds in water suspension.

Two-tenths ml. of each of the 3,000 ppm suspensions of each of Compound Nos. 1-37 was pipetted onto the surface of 5 g. of a synthetic diet mixture held in partially filled cells of a plastic jelly tray. Five cells of each of the compounds at a 3,000 ppm concentration were so treated. Following this treatment a second instar larva of the tobacco budworm, *Heliothis virescens*, was placed in each of the so-treated cells. In addition, the same larva of the tobacco budworm was disposed in five additional cells of a plastic jelly tray in which each cell was provided with 5 g. of a synthetic diet mixture. In these additional 5 cells no treatment with the test formulations was provided. Thus, a comparison between the test cells and the control cells was obtained to determine the percent control of Compound Nos. 1-33.

At the end of one and two weeks, the trays were examined and the percent control determined. The results of this test are summarized in Table II.

EXAMPLE 7

Control of Southern Corn Rootworm

Suspensions of Compound Nos. 1-37 were obtained by the method set forth in Example 6 except that the amount of water added was increased to obtain the more dilute concentration of 500 ppm for the suspensions containing the compounds of this invention.

Five ml. of 500 ppm concentration suspensions of each of Compound Nos. 1-37 were pipetted onto a paper towel. The towel was inserted into a Ziploc [trademark] plastic bag. Two corn seedlings, also soaked in each of the 500 ppm suspensions of Compound Nos. 1-37, were placed in each of the plastic bags. The bags were closed and held in that condition for 18 hours. At that time each of the bags was loaded with 5 corn rootworm, Diabrotica undecimpunctata, larvae. After 6 days the number of live larvae were counted. The percent control was calculated by comparing the number of live larvae with the number remaining in the controls which were also provided with corn seedlings disposed in Ziploc [trademark] plastic bags and similarly infected with 5 corn rootworms 18 hours after introduction of the corn seedlings therein. However, in the case of the controls, paper towels were not coated with the suspensions the compounds of the present invention nor were the corn seedlings soaked in these suspensions.

The result of this test is summarized in Table II.

EXAMPLE 8

Control of Rice Planthopper

Formulations of each of Compound Nos. 1-37 in suspension concentration of 1,000 ppm were made in accordance with the procedure of Example 6 except that the amount of water was increased to lower the concentration of the active compound to 1,000 ppm.

Two rice seedling plants were treated with each of these suspensions by spraying the suspensions onto the seedling plants with a spray atomizer. Controls were prepared wherein the seedling plants were not treated with the suspensions of the compounds of the present invention. Ten adult rice planthoppers, Sogatodes cryzicola, were placed on each of the treated and untreated plants one day following treatment with the compounds of the present invention. The surviving planthoppers were counted after 5 days in the treated and untreated control plants to determine percent control of Compounds No. 1-37.

The results of this test is summarized in Table II.

EXAMPLE 9

Control of Mites

As in Example 8, suspensions at a concentration of 1,000 ppm of Compound Nos. 1-37 were prepared. The plant cowpeas, in the first primary leaf stage, was used in this test. Two plants per pot, one primary leaf each, were used for each replicate. Two replicates were used for each compound tested. The plants were sprayed with dispersions of each of Compound Nos. 1-37 using a spray atomizer to thoroughly drench the foliage.

One day following this treatment, a circle of tangelfoot was placed on the upper surface of the treated leaves and adult mites, Tetranychus urticae Koch, were transferred into this confinement.

Six days following infestation with the mites, the plants were examined for adult live mites remaining on the leaves. On an estimated basis, in comparison with the number of living mites on the control plants, which were prepared in the same way as the treated plants except for the treatment with the suspensions of the compounds of the present invention, the percent control was determined.

The results of this test are summarized in Table II below.

TABLE II

Percent Control of Pests

| Cpd. No. | TB[a] | CR[b] | RP[c] | MI[d] |
|---|---|---|---|---|
| 1 | 100 | 0 | 0 | 0 |
| 2 | 100 | 100 | 0 | 85 |
| 3 | 80 | 20 | 0 | 60 |
| 4 | 60 | 0 | 0 | 60 |
| 5 | 60 | 20 | 0 | 0 |
| 6 | 80 | 0 | 0 | 0 |
| 7 | 100 | 0 | 98 | 80 |
| 8 | 80 | 37 | 0 | 0 |
| 9 | 20 | 37 | 50 | 60 |
| 10 | 0 | 56 | 0 | 50 |
| 11 | 80 | 0 | 0 | 60 |
| 12 | 0 | 20 | 0 | 0 |
| 13 | 100 | 100 | 0 | 100 |
| 14 | 100 | 80 | 100 | 100 |
| 15 | 100 | 73 | 0 | 90 |
| 16 | 80 | 11 | 0 | 50 |
| 17 | 100 | 78 | 0 | 100 |
| 18 | 100 | 100 | 0 | 80 |
| 19 | 0 | 20 | 0 | 0 |
| 20 | 0 | 79 | 0 | 80 |
| 21 | 0 | 40 | 0 | 0 |
| 22 | 100 | 56 | 95 | 70 |
| 23 | 100 | 60 | 100 | 80 |
| 24 | 100 | 100 | 80 | 0 |
| 25 | 100 | 20 | 0 | 0 |
| 26 | 100 | 37 | 0 | 30 |
| 27 | 40 | 0 | 0 | 0 |

TABLE II (Continued)

| Cpd. No. | TB[a] | CR[b] | RP[c] | MI[d] |
|---|---|---|---|---|
| 28 | 40 | 16 | 0 | 0 |
| 29 | 100 | 79 | 100 | 90 |
| 30 | 20 | 16 | 0 | 0 |
| 31 | 0 | 20 | 0 | 0 |
| 32 | 0 | 40 | 0 | 0 |
| 33 | 80* | 0 | 0 | 0 |
| 34 | 79 | 100 | 0 | 0 |
| 35 | 100 | 40 | 100 | 100 |
| 36 | 100 | 100 | 0 | 100 |
| 37 | 33 | 100 | 0 | 98 |

Footnotes

a - Tobacco Budworm

b - Corn Rootworm

c - Rice Planthopper

d - Mites

* Treated with a suspension having a concentration of 16,000 ppm.

EXAMPLE 10

Control of Nematodes

The Southern root-knot nematode *Meloidogyne incognita* was reared in sandy, cultured soil using tomato as host plant. Roots from the thus nematode-cultured plants were ground in a Waring [trademark] blender. Ground roots and culture soil were mixed with equal parts of uninfested soil. This mixture was placed in pots.

Dispersions were prepared using 5 compounds of the present invention, Compound Nos. 7, 20, 23, 24 and 27, at a concentration of 1,000 ppm in accordance with the procedure set forth in Example 6. Each of these dispersions were added to the above-described pots by drenching each pot with 25 ml. of the dispersion to provide a resultant soil concentration of 50 ppm of each of the compounds tested.

One day after treatment with the 50 ppm dispersions, two tomato seedlings were planted in each pot. Nineteen days after planting, the roots were evaluated by washing away the soil and comparing the number of knots on plant roots on the treated soil with the number of knots noted on the untreated nematode-infested controls. This comparison yielded the percent control.

It is emphasized that the controls were prepared and grown identically with the treated pots except the control pots were not treated with a dispersion of any of Compound Nos. 7, 20, 23, 24 or 27.

The results of this nematode soil test are summarized below in Table III.

TABLE III

| Cpd. No. | % Nematode Control |
|---|---|
| 7 | 40 |
| 20 | 30 |
| 23 | 80 |
| 24 | 70 |
| 27 | 50 |

The above embodiments and examples illustrate the scope and spirit of the instant invention. These embodiments and examples will make apparent, to those skilled in the art, other embodiments and examples. These other embodiments and examples are within the contemplation of the present invention. Therefore, the instant invention should be limited only to the appendix claims.

## Claims

1. A compound having the structural formula

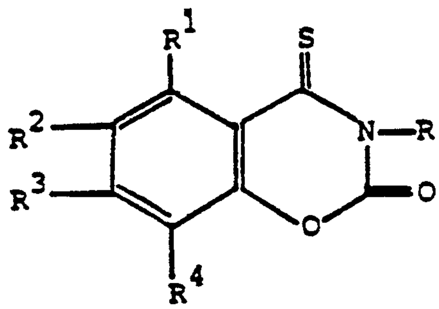

where R is $C_1$-$C_6$ alkyl or $C_2$-$C_6$ alkenyl; $R^1$ is hydrogen or $C_1$-$C_6$ alkyl; $R^2$ is hydrogen, $C_1$-$C_6$ alkyl, $C_1$-$C_6$ alkoxy, halogen, alkylthio, $C_3$-$C_6$ cycloalkyl, $C_7$-$C_9$ aralkyl, $C_7$-$C_9$ aralkyloxy, carboxy or $C_2$-$C_5$ carbalkoxy; $R^3$ is hydrogen, $C_1$-$C_6$ alkyl, $C_1$-$C_6$ alkoxy, halogen, $C_6$-$C_{10}$ aryl or $C_1$-$C_2$ haloalkyl; and $R^4$ is hydrogen, $C_1$-$C_6$ alkyl, $C_1$-$C_6$ alkoxy, halogen, $C_7$-$C_9$ aralkyl or $C_7$-$C_9$ aralkyloxy with the proviso that R cannot be methyl if $R^1$, $R^2$, $R^3$ and $R^4$ are each hydrogen.

2. A compound in accordance with Claim 1 wherein R is $C_1$-$C_4$ alkyl or $C_2$-$C_4$ alkenyl; $R^1$ is hydrogen or $C_1$-$C_4$ alkyl; $R^2$ is hydrogen, $C_1$-$C_4$ alkyl, $C_2$-$C_4$ carbalkoxy, $C_1$-$C_4$ alkoxy or halogen; $R^3$ is hydrogen, $C_1$-$C_4$ alkyl, $C_1$-$C_4$ alkoxy or trifluoromethyl; and $R^4$ is hydrogen, $C_1$-$C_4$ alkyl, $C_1$-$C_4$ alkoxy, $C_7$-$C_8$ aralkyl or halogen with the proviso that R cannot be methyl when $R^1$, $R^2$, $R^3$ and $R^4$ are each hydrogen.

3. A compound in accordance with Claim 2 wherein R is $C_1$-$C_2$ alkyl; $R^1$ is hydrogen; $R^2$ is hydrogen or methyl; $R^3$ is hydrogen, $C_1$-$C_3$ alkyl or $C_1$-$C_3$ alkoxy; and $R^4$ is hydrogen or $C_1$-$C_4$ alkyl with the proviso that R cannot be methyl if $R^1$, $R^2$, $R^3$ and $R^4$ are each hydrogen.

4. A process for making a compound having the structural formula

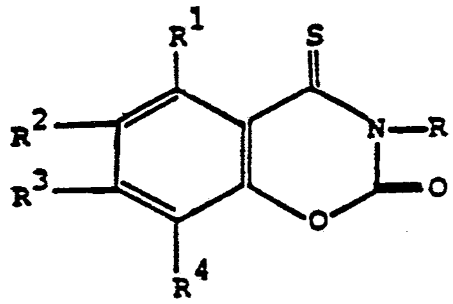

where R is $C_1$-$C_6$ alkyl or $C_2$-$C_6$ alkenyl; $R^1$ hydrogen or $C_1$-$C_6$ alkyl; $R^2$ is hydrogen, $C_1$-$C_6$ alkyl, $C_1$-$C_6$ alkoxy, halogen, alkylthio, $C_3$-$C_6$ cycloalkyl, $C_7$-$C_9$ aralkyl, $C_7$-$C_9$ aralkyloxy, carboxy or $C_2$-$C_5$ carbalkoxy; $R^3$ is hydrogen, $C_1$-$C_6$ alkyl, $C_1$-$C_6$ alkoxy, halogen, $C_6$-$C_{10}$ aryl or $C_1$-$C_2$ haloalkyl; and $R^4$ is hydrogen, $C_1$-$C_6$ alkyl, $C_1$-$C_6$ alkoxy, halogen, $C_7$-$C_9$ aralkyl or $C_7$-$C_9$ aralkyloxy comprising reacting, at a temperature of up to 100°C and in the presence of an organic base, a 2-hydroxybenzenecarbodithioate ester compound having the structural formula:

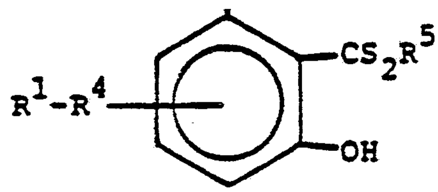

where $R^5$ is $C_1$-$C_6$ alkyl; and $R^1$, $R^2$, $R^3$ and $R^4$ have the meanings given above with a compound having the structural formula RNCO where R has the meanings given above.

5. A process in accordance with Claim 4 wherein said organic base is selected from the group consisting of triethylamine and pyridine.

6. A process for controlling plant pests comprising applying to the locus to be protected a pesticidally effective amount of a compound having the structural formula

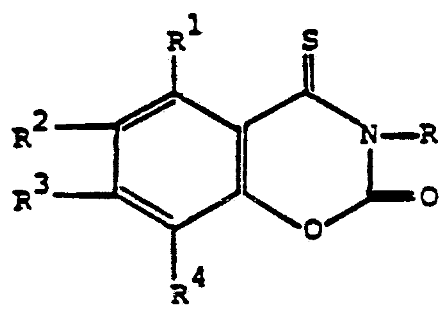

where R is $C_1$-$C_6$ alkyl or $C_2$-$C_6$ alkenyl; $R^1$ is hydrogen or $C_1$-$C_6$ alkyl; $R^2$ is hydrogen, $C_1$-$C_6$ alkyl, $C_1$-$C_6$ alkoxy, halogen, alkylthio, $C_3$-$C_6$ cycloalkyl, $C_7$-$C_9$ aralkyl, $C_7$-$C_9$ aralkyloxy, carbalkoxy or $C_2$-$C_5$ carbalkoxy; $R^3$ is hydrogen, $C_1$-$C_6$ alkyl, $C_1$-$C_6$ alkoxy, halogen, $C_6$-$C_{10}$ aryl or $C_1$-$C_2$ haloalkyl; $R^4$ is hydrogen, $C_1$-$C_6$ alkyl, $C_1$-$C_6$ alkoxy, halogen, $C_7$-$C_9$ aralkyl or $C_7$-$C_9$ aralkyloxy.

7. A process in accordance with Claim 6 wherein R is $C_1$-$C_4$ alkyl or $C_1$-$C_4$ alkenyl; $R^1$ is hydrogen or $C_1$-$C_4$ alkyl; $R^2$ is hydrogen, $C_1$-$C_4$ alkyl, $C_2$-$C_4$ carbalkoxy, $C_1$-$C_4$ alkoxy or halogen; $R^3$ is hydrogen, $C_1$-$C_4$ alkyl, $C_1$-$C_4$ alkoxy or trifluoromethyl; and $R^4$ is hydrogen, $C_1$-$C_4$ alkyl, $C_1$-$C_4$ alkoxy, $C_7$-$C_8$ aralkyl or halogen.

8. A process in accordance with Claim 7 wherein R is $C_1$-$C_2$ alkyl; $R^1$ is hydrogen; $R^2$ is hydrogen or methyl; $R^3$ is hydrogen, $C_1$-$C_3$ alkyl or $C_1$-$C_3$ alkoxy; and $R^4$ is hydrogen or $C_1$-$C_4$ alkyl.

9. A process in accordance with Claim 6 wherein said pest is an insect, an arachnid or a nematode.

10. A pesticidal composition comprising a compound having the structural formula

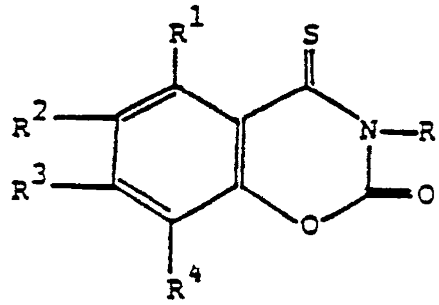

where R is $C_1$-$C_6$ alkyl or $C_2$-$C_6$ alkenyl; $R^1$ is hydrogen or $C_1$-$C_6$ alkkyl; $R^2$ is hydrogen, $C_1$-$C_5$ alkyl, $C_1$-$C_6$ alkoxy, halogen, alkylthio, $C_3$-$C_6$ cycloalkyl, $C_7$-$C_9$ aralkyl, $C_7$-$C_9$ aralkyloxy, carboxy or $C_2$-$C_5$ carbalkoxy; $R^3$ is hydrogen, $C_1$-$C_6$ alkyl, $C_1$-$C_6$ alkoxy, halogen, $C_6$-$C_{10}$ aryl or $C_1$-$C_6$ haloalkyl; $R^4$ is hydrogen, $C_1$-$C_6$ alkyl, $C_1$-$C_6$ alkoxy, halogen, $C_7$-$C_9$ aralkyl or $C_7$-$C_9$ aralkyloxy; and a carrier therefor.

11. A composition in accordance with Claim 10 wherein R is $C_1$-$C_4$ alkyl or $C_2$-$C_4$ alkenyl; $R^1$ is hydrogen or $C_1$-$C_4$ alkyl; $R^2$ is hydrogen, $C_1$-$C_4$ alkyl, $C_2$-$C_4$ carbalkoxy, $C_1$-$C_4$ alkoxy or halogen; $R^3$ is hydrogen, $C_1$-$C_4$ alkyl, $C_1$-$C_4$ alkoxy or trifluoromethyl; and $R_4$ is hydrogen, $C_1$-$C_4$ alkyl, $C_1$-$C_4$ alkoxy, $C_7$-$C_8$ aralkyl or halogen.

12. A composition in accordance with Claim 11 wherein R is $C_1$-$C_2$ alkyl; $R^1$ is hydrogen; $R^2$ is hydrogen or methyl; $R^3$ is hydrogen, $C_1$-$C_3$ alkyl or $C_1$-$C_3$ alkoxy; and $R^4$ is hydrogen or $C_1$-$C_4$ alkyl.

13. A composition in accordance with any of claims 10 to 12 wherein said composition is employed to effectively control insects, arachnids or nematodes.

European Patent Office

# EUROPEAN SEARCH REPORT

Application number

EP 88303341.7

## DOCUMENTS CONSIDERED TO BE RELEVANT

| Category | Citation of document with indication, where appropriate, of relevant passages | Relevant to claim | CLASSIFICATION OF THE APPLICATION (Int. Cl 4) |
|---|---|---|---|
| A | DE - C2 - 1 793 795 (BAYER AG) * Totality * | 1,4,6, 9,10, 13 | C 07 D 265/24<br>A 01 N 43/86 |
| A | DE - C2 - 1 793 781 (BAYER AG) * Totality * | 1,4,6, 9,10, 13 | |
| A | FR - A - 1 522 005 (FARBENFABRIKEN BAYER) * Claims * | 1,4,6, 9,10, 13 | |
| A | CHEMICAL ABSTRACTS, vol. 83, no. 13, September 29, 1975, Columbus, Ohio, USA<br>PALAZZO, S.; GIANNOLA, L.I.; CARONNA, S. "Synthesis of sulfur derivatives of benzoxazine and benzothiazine"<br>page 550, column 2, abstract-no. 114 318d<br>& Atti Accad.Sci., Lett.Arti Palermo, Parte 1 1973 (Pub. 1974), 33(2), 421-7 | 1,4 | TECHNICAL FIELDS SEARCHED (Int Cl 4)<br>C 07 D 265/00 |

The present search report has been drawn up for all claims

| Place of search | Date of completion of the search | Examiner |
|---|---|---|
| VIENNA | 06-07-1988 | BRUS |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document